# EUROPEAN PATENT APPLICATION

(11) **EP 2 345 734 A2**
(43) Date of publication of application: **20.07.2011**
(21) Application number: 10174944.8
(22) Date of filing: 20.02.1998
(51) Int. Cl.: C12P 17/06, C12N 15/61, C12N 15/55, C12P 37/00, C12P 35/00, C12N 9/16, C12N 9/92, C12P 17/18, C12P 23/00, C12P 7/64, C12N 9/26, C12N 15/01, C12P 19/62

(54) **Fermentative production of valuable compounds on an industrial scale using chemically defined media**

(30) Priority: 20.02.1997 EP 97200494
(62) Divisional of application: 06114041.4
(71) Applicant: DSM IP Assets B.V., 6411 TE Heerlen (NL)
(72) Inventor: Koekman, Bertus Pieter, 2636 BG Schipluiden (NL); de Laat, Wilhelmus Theodorus Antonius Maria, 4817 KW Breda (NL); Preusting, Johannes Cornelis Gerardus, 9722 WD Groningen (NL)
(74) Representative: Elkenbracht, Johan Christiaan

(57) **Abstract**

The present invention describes the use of chemically defined media for the fermentative production of valuable compounds on an industrial scale. Microbial strains which are suitable for fermentation on an industrial scale using a chemically defined medium include fungal, yeast and bacterial strains. Suitable strains can be obtained as wild type strains or by screening and selection after mutagenic treatment or DNA transformation.

## Description

### Field of the invention

The present invention relates to the field of fermentation, i.e. the fermentative production of valuable compounds, such as primary or secondary metabolites, pharmaceutical proteins or peptides, or industrial enzymes.

### Background of the invention

Many valuable compounds are manufactured by fermentative production in large, industrial scale fermentors, i.e. the microorganism which produces a valuable compound of interest is grown under controlled conditions in a fermentor of 10 to 300 m³. In current industrial scale fermentation processes, the production organism typically is fermented in a complex fermentation medium. A complex medium is understood to be a medium comprising a complex nitrogen and/or carbon source, such as soybean meal, cotton seed meal, corn steep liquor, yeast extract, casein hydrolysate, molasses, and the like.

Advantages of complex media are that the constituent complex raw materials are not expensive, readily available and form a complete or nearly complete nutrient source for the microorganism, containing a carbon and nitrogen source as well as vitamins and minerals. Furthermore, the mixture of biological macromolecules as present in complex raw materials, like proteins, carbohydrates, lipids, and the like, need to be degraded by enzymes excreted by the microorganism prior to their consumption. As a consequence, consumable small molecules become available evenly throughout the fermentor and during the fermentation process, thereby avoiding concentration gradients and mixing problems and keeping the level of these consumable small molecules below repressing concentrations. Furthermore, these macromolecules as well as organic acids also present in complex media give the medium a buffering capacity, in this way facilitating pH control.

In addition to these advantages, complex fermentation media have several important disadvantages. Most importantly, complex raw materials have a chemically undefined composition and an variable quality, a.o. due to seasonal variation and different geographical origin. Since the composition of fermentation media has an important influence on fermentation parameters like viscosity, heat transfer and oxygen transfer, complex raw materials are a major cause of process variability. In addition, they hamper downstream processing and may adversely influence the quality of the final product. For instance, fermentation broths, in particular of filamentous microorganisms, may display a decreased filterability when using complex raw materials.

Complex raw materials may also contain compounds which unintentionally accumulate in or are coisolated with the end product. Heavy metals, pesticides or herbicides are examples of undesirable compounds which may be present in complex raw materials. Moreover, complex raw materials may contain or may lead to the formation of toxins.

Further disadvantages are that complex media generate an unfavourable smell during sterilization and produce undesirable waste streams.

Despite the above-identified disadvantages associated with the use of complex media, these media still are preferred for large scale industrial fermentation processes. There are various reasons why media not containing complex raw materials, i.e. chemically defined media, have not been considered for use in industrial scale fermentation processes. One obvious reason is found in the advantages associated with the use of complex media. More importantly, the product yields which would be obtained using chemically defined media on an industrial scale typically were considered to be substantially lower than those obtained using media containing complex raw materials. In addition, high-producing microbial strains which have been developed for industrial processes in complex media may not retain their good performance in chemically defined media. One reason for an unsatisfactory performance in a chemically defined medium may be that current industrial strains have undergone various rounds of mutagenesis and selection, without considering their performance on chemically defined media.

Chemically defined media thus far have been applied for research purposes only, i.e. in laboratory cultures in petri dishes and/or shake flasks or on a relatively small fermentative scale typically not exceeding a volume of about 20-40 L. See for instance the fermentative production of secondary metabolites, such as penicillin (Jarvis and Johnson, J. Am. Chem. Soc. 69, 3010-3017 (1947); Stone, and Farrell, Science 104, 445-446 (1946); White et al., Arch. Biochem. 8, 303-309 (1945)), clavulanic acid (Romero et al., Appl. Env. Microbiol. 52, 892-897 (1986) and erythromycin (Bushell et al., Microbiol. 143, 475-480 (1997)).

However, investigations regarding the use of chemically defined media on such small research scales do not provide any teaching to the person skilled in the art regarding the applicability of these media in large scale industrial fermentations processes for production purposes, typically having a volume scale of about 10 m³ or larger.

To avoid the problems associated with the use of conventional recipes for complex media in current industrial practice, it would be desirable to apply chemically defined recipes for industrial scale fermentations.

Here, we describe the use of chemically defined media for industrial scale fermentation processes, allowing - in combination with a suitable strain - the production of valuable compounds, such as primary or secondary metabolites, pharmaceutical proteins or peptides, or industrial enzymes, in an economically attractive yield.

### Summary of the invention

The present invention discloses an industrial process for the production of a valuable compound, comprising the steps of fermentation of a microbial strain in a fermentation medium which is a chemically defined medium essentially composed of chemically defined constituents and recovery of the valuable compound from the fermentation broth.

The present invention further discloses a method for preparing and/or improving a microbial strain producing a valuable compound of interest which is capable of being fermented on an industrial scale in a chemically defined medium, comprising the steps of:
* subjecting a suitable parent strain to a mutagenic treatment selected from the group of physical means and chemical mutagens, and/or to DNA transformation,
* screening the resulting mutants and/or transformants for their growth performance on a chemically defined medium and their production level of said valuable compound of interest,
* selecting mutants having a similar or improved growth performance on a chemically defined medium and/or an improved production level of said valuable compound of interest as compared to said parent strain.

### Detailed description of the invention

The present invention describes the use of chemically defined fermentation media for the industrial scale fermentation of a suitable microbial strain, said suitable microbial strain being capable of production of a valuable compound.

Throughout the description of the invention, an industrial scale fermentation process or an industrial process is understood to encompass a fermentation process on a volume scale which is ≥ 10 m³, preferably ≥ 25 m³, more preferably ≥ 50 m³, most preferably ≥ 100 m³.

The term "chemically defined" is understood to be used for fermentation media which are essentially composed of chemically defined constituents. A fermentation medium which is essentially composed of chemically defined constituents includes a medium which does not contain a complex carbon and/or nitrogen source, i.e. which does ot contain complex raw materials having a chemically undefined composition. A fermentation medium which is essentially composed of chemically defined constituents may further include a medium which comprises an essentially small amount of a complex nitrogen and/or carbon source, an amount as defined below, which typically is not sufficient to maintain growth of the microorganism and/or to guarantee formation of a sufficient amount of biomass.

In that regard, complex raw materials have a chemically undefined composition due to the fact that, for instance, these raw materials contain many different compounds, among which complex heteropolymeric compounds, and have a variable composition due to seasonal variation and differences in geographical origin. Typical examples of complex raw materials functioning as a complex carbon and/or nitrogen source in fermentation are soybean meal, cotton seed meal, corn steep liquor, yeast extract, casein hydrolysate, molasses, and the like.

An essentially small amount of a complex carbon and/or nitrogen source may be present in the chemically defined medium according to the invention, for instance as carry-over from the inoculum for the main fermentation. The inoculum for the main fermentation is not necessarily obtained by fermentation on a chemically defined medium. Most often, carry-over from the inoculum will be detectable through the presence of a small amount of a complex nitrogen source in the chemically defined medium for the main fermentation.

It may be advantageous to use a complex carbon and/or nitrogen source in the fermentation process of the inoculum for the main fermentation, for instance to speed up the formation of biomass, i.e. to increase the growth rate of the microorganism, and/or to facilitate internal pH control. For the same reason, it may be advantageous to add an essentially small amount of a complex carbon and/or nitrogen source, e.g. yeast extract, to the initial stage of the main fermentation, especially to speed up biomass formation in the early stage of the fermentation process.

An essentially small amount of a complex carbon and/or nitrogen source which may be present in the chemically defined medium according to the invention is defined to be an amount of at the most about 10% of the total amount of carbon and/or nitrogen (Kjeldahl N) which is present in the chemically defined medium, preferably an amount of at the most 5% of the total amount of carbon and/or nitrogen, more preferably an amount of at the most 1% of the total amount of carbon and/or nitrogen. Most preferably, no complex carbon and/or nitrogen source is present in the chemically defined medium according to the invention.

It is to be understood that the term "chemically defined medium" as used in the present invention includes a medium wherein all necessary components are added to the medium before the start of the fermentation process, and further includes a medium wherein part of the necessary components are added before starting and part are added to the medium during the fermentation process.

The present invention further discloses that microbial strains are able to convert, on an industrial scale, the simple raw materials of chemically defined media into an economically attractive amount of valuable product. It is surprisingly found that the productivity of microbial strains in chemically defined media, when measured on an industrial scale, may be comparable to or in some cases even higher than their productivity in complex media.

A further advantage of the use of chemically defined media is that the oxygen transfer from the gas phase to the liquid phase and the carbon dioxide transfer from the liquid phase to the gas phase is improved substantially as compared to using complex media. As known to those skilled in the art, dissolved oxygen and dissolved carbon dioxide concentrations are two important factors in scale up of a fermentation process, and can determine the economical feasibility of an industrial process. The improved mass transfer obtained using chemically defined media can be attributed to the absence in these media of substantial amounts of compounds which promote coalescence of gas bubbles. Coalescence-promoting compounds for instance can be found among certain hydrophobic and/or polymeric compounds present in complex raw materials. Coalescence of gass bubbles typically results in a lower mass transfer coefficient (van't Riet and Tramper, in: Basic Bioreactor Design, pp 236-273 (1991)).

Oxygen transfer often is a limiting factor in fermentation processes, especially in fermentations of filamentous microorganisms. The improved oxygen transfer capacity obtained when fermentation is performed using a chemically defined medium according to the invention provides a much cheaper way of optimization of the productivity than investments in hardware, like power input, oxygen enrichment of the inlet air or fermentor pressure.

In industrial fermentation processes, filamentous microorganisms, like filamentous bacteria such as Actinomycetes or filamentous fungi such as Penicillium or Aspergillus, typically are grown having a pellet morphology. In that regard, proteins and peptides present in complex fermentation media have the tendency to produce fluffy pellets, which easily fall apart to dispersed mycelium with very long and branched hyphae as a consequence of the high growth rates which typically are obtained using complex media. Therefore, a fluffy pellet morphology generally may cause a undesirably high broth viscosity. The use of chemically defined media has a favorable influence on morphology, for instance by producing a more rigid pellet which does not easily fall apart during fermentation. In this way, a significant decrease of the viscosity of filamentous fermentation broths may be obtained using chemically defined media. Since a low viscosity of the fermentation broth is advantageous for product formation, control of viscosity is of the utmost importance in industrial scale fermentation processes.

Another advantage of the use of chemically defined media is found in downstream processing of the product. For certain strain-product combinations, especially when filamentous strains are fermented, downstream processing is significantly improved by using chemically defined media.

A chemically defined medium to be used in the process of the invention typically should contain the so-called structural and the so-called catalytic elements.

Structural elements are those elements which are constituents of microbial macromolecules, i.e. hydrogen, oxygen, carbon, nitrogen, phosphorus and sulphur. The structural elements hydrogen, oxygen, carbon and nitrogen typically are contained within the carbon and nitrogen source. Phosphorus and sulphur typically are added as phosphate and sulphate and/or thiosulphate ions.

The type of carbon and nitrogen source which is used in the chemically defined medium is not critical to the invention, provided that the carbon and nitrogen source have essentially a chemically defined character.

Preferably, a carbon source is selected from the group consisting of carbohydrates such as glucose, lactose, fructose, sucrose, maltodextrins, starch and inulin, glycerol, vegetable oils, hydrocarbons, alcohols such as methanol and ethanol, organic acids such as acetate and higher alkanoic acids. More preferably, a carbon source is selected from the group consisting of glucose, sucrose and soybean oil. Most preferably, the carbon source is glucose. It is to be understood that the term "glucose" includes glucose syrups, i.e. glucose compositions containing glucose oligomers in defined amounts.

A nitrogen source preferably is selected from the group consisting of urea, ammonia, nitrate, ammonium salts such as ammonium sulphate, ammonium phosphate and ammonium nitrate, and amino acids such as glutamate and lysine. More preferably, a nitrogen source is selected from the group consisting of ammonia, ammonium sulphate and ammonium phosphate. Most preferably, the nitrogen source is ammonia. The use of ammonia as a nitrogen source has the advantage that ammonia additionally can function as a pH-controlling agent. In case ammonium sulphate and/or ammonium phosphate are used as a nitrogen source, part or all of the sulphur and/or phosphorus requirement of the microorganism may be met.

Catalytic elements are those elements which are constituents of enzymes or enzyme cofactors. These elements are for instance magnesium, iron, copper, calcium, manganese, zinc, cobalt, molybdenum, selenium, borium.

Next to these structural and catalytic elements, cations such as potassium and sodium ions should be present to function as a counter ion and for control of intracellular pH and osmolarity.

Compounds which may optionally be included in a chemically defined medium are chelating agents, such as citric acid, and buffering agents such as mono- and dipotassium phosphate, calcium carbonate, and the like. Buffering agents preferably are added when dealing with processes without an external pH control. In addition, an antifoaming agent may be dosed prior to and/or during the fermentation process.

Macromolecules and organic acids which are present in complex media provide for a buffering capacity in these media. Due to the absence of these compounds in chemically defined media, pH control is more difficult in chemically defined than in complex media. The present invention shows that a pH control wherein either an acid or a base may be dosed, depending on the pH development in the broth, allows for a proper pH profile in a chemically defined industrial scale process.

Vitamins refer to a group of structurally unrelated organic compounds which are necessary for the normal metabolism of microorganisms. Microorganisms are known to vary widely in their ability to synthesize the vitamins they require. A vitamin should be added to the fermentation medium of a microorganism not capable to synthesize said vitamin. Typically, chemically defined fermentation media for yeasts or bacteria or for certain lower fungi, e.g. Mucorales, may be supplemented with one or more vitamin(s). Higher fungi most often have no vitamin requirement.

Vitamins are selected from the group of thiamin, riboflavin, pyridoxal, nicotinic acid or nicotinamide, pantothenic acid, cyanocobalamin, folic acid, biotin, lipoic acid, purines, pyrimidines, inositol, choline and hemins.

Structural and catalytic elements and, optionally, vitamins are necessary for growth of the microorganism, i.e. for biomass formation.

The amount of necessary compounds, i.e. compounds comprising structural and catalytic elements and, optionally, vitamins, to be added to the chemically defined medium will mainly depend on the amount of biomass which is to be formed in the fermentation process. The amount of biomass formed may vary widely, typically from about 10 to about 150 g/l fermentation broth. In general, fermentations producing an amount of biomass which is lower than about 10 g/l are not industrially relevant.

In addition, the optimum amount of each constituent of a defined medium, as well as which compounds are essential and which are non-essential, will depend on the type of microorganism which is subjected to fermentation in a defined medium, on the amount of biomass and on the product to be formed. The use of chemically defined media thereby advantageously allows for a variation of the concentration of each medium component independently from the other components, in this way facilitating optimization of the medioum composition.

For product formation, it may be necessary to supplement the chemically defined medium with additional compounds and/or to increase the concentration of certain compounds already present in the chemically defined medium above the level which is necessary for growth of the microorganism. The function of the said compounds may be that they induce and/or enhance the production of a desired compound by the microorganism, or that they function as a precursor for a desired compound.

Examples of compounds to be supplemented and/or to be added in an increased amount to a chemically defined medium are: sulphate in an increased amount for the production of β-lactam compounds, nitrogen-containing compounds in an increased amount for the production of amino acids, especially basic amino acids, phenylacetic acid for the production of penicillin G, phenoxyacetic acid for the production of penicillin V, adipic acid for the production of adipyl-7-ADCA and adipyl-7-ACA, propionic acid for the production of erythromycin.

In an industrial fermentation process according to the invention, the total amount of carbon source added to the chemically defined medium, expressed as amount of carbon / liter medium, may vary from 10 to 200 g C/I preferably from 20 to 200 g C/I.

The total amount of nitrogen source added to the chemically defined medium may vary from 0.5 to 50 g N/I, preferably from 1 to 25 g N/I, wherein N is expressed as Kjeldahl nitrogen.

The ratio between carbon and nitrogen source in a fermentation may vary considerably, whereby one determinant for an optimal ratio between carbon and nitrogen source is the elemental composition of the product to be formed.

Additional compounds required for growth of a microorganism, like phosphate, sulphate or trace elements, are to be added using the concentration ranges as indicated in Table 1 as a guideline. The concentration ranges of these additional compounds may vary between different classes of microorganisms, i.e. between fungi, yeasts and bacteria.

Vitamin concentrations generally fall within the range of 0.1 (biotin) to 500 (myo-inositol) mg/l.

Typically, the amount of medium components necessary for growth of a microorganism may be determined in relation to the amount of carbon source used in the fermentation, since the amount of biomass formed will be primarily determined by the amount of carbon source used.

**Table 1. Typical concentration ranges of medium components, besides the carbon and nitrogen source, necessary for growth of various classes of microorganisms (g/l).**

| | fungi | yeasts | bacteria (Actinomycetes) |
|---|---|---|---|
| PO₄^{1,5} | 1-20 | | |
| SO₄^{2,5} | | | |
| MgSO₄.7aq³ | 0.5-10 | 0.5-2 | 0.5-2 |
| CaCl₂.2aq³ | 0.01-0.1 | 0.1-1 | 0.05-0.5 |
| FeSO₄.7aq³ | 0.1-1.0 | 0.1-0.5 | 0.1-0.5 |
| ZnSO₄.7aq³ | 0.0005-0.1 | 0.002-1 | 0.002-0.1 |
| MnSO₄.1aq³ | 0.0005-0.1 | 0.002-1 | 0.002-0.1 |
| CuSO₄.5aq^{3,4} | ≤ 0.005 | 0.001-0.01 | 0.001-0.01 |
| CoSO₄.7aq^{3,4} | ≤ 0.01 | ≤ 0.01 | ≤ 0.01 |
| Na₂MoO₄.2aq⁴ | ≤ 0.0005 | 0.001-0.005 | 0.001-0.005 |
| H₃BO₃⁴ | ≤ 0.0005 | 0.001-0.005 | 0.001-0.005 |
| Kl⁴ | | ≤ 0.002 | ≤ 0.002 |

| | | | |
|---|---|---|---|
| ¹ The basal amount of phosphate needed will be 0.5-1% of the biomass dry weight. For batch processes on relatively small scale, extra phosphate will be required for pH control. ² Sulphate may also be dosed via the titrant, like K⁺ and Na⁺. ³ Sulphate may be (partially) replaced by chloride as a counter ion in the trace elements, or *vice versa*. ⁴ For some trace elements lower limits are difficult to define. Their requirement may for instance be met by their presence in other medium components, e.g. ferrous sulphate, water, small amounts of yeast extract, etc. ⁵ Phosphate and sulphate are added as potassium, ammonium and/or sodium salts, with a preference of K > NH₄ > Na. | | | |

An industrial fermentation process according to the invention using a chemically defined medium can be performed as a batch, a repeated batch, a fed-batch, a repeated fed-batch or a continuous fermentation process.

In a batch process, all medium components are added directly, as a whole, to the medium before the start of the fermentation process.

In a repeated batch process, a partial harvest of the broth accompanied by a partial supplementation of complete medium occurs, optionally repeated several times.

In a fed-batch process, either none or part of the compounds comprising one or more of the structural and/or catalytic elements is added to the medium before the start of the fermentation and either all or the remaining part, respectively, of the compounds comprising one or more of the structural and/or catalytic elements is fed during the fermentation process. The compounds which are selected for feeding can be fed together or separate from each other to the fermentation process.

Especially in a fermentation process wherein the original fermentation medium is diluted about two times or more by a feed of compounds comprising one or more of the structural elements, the feed may further comprise catalytic elements and additional medium components, next to the structural elements.

In a repeated fed-batch or a continuous fermentation process, the complete start medium is additionally fed during fermentation. The start medium can be fed together with or separate from the structural element feed(s). In a repeated fed-batch process, part of the fermentation broth comprising the biomass is removed at regular time intervals, whereas in a continuous process, the removal of part of the fermentation broth occurs continuously. The fermentation process is thereby replenished with a portion of fresh medium corresponding to the amount of withdrawn fermentation broth.

In a preferred embodiment of the invention, a fed-batch or repeated fed-batch process is applied, wherein the carbon and/or the nitrogen source and/or phosphate are fed to the fermentation process. In a more preferred embodiment, the carbon and nitrogen source are fed to the fermentation process. Most preferably, the carbon and nitrogen source, as well as phosphate are fed. In that regard, a preferred carbon source is glucose and a preferred nitrogen source is ammonia and/or ammonium salts.

The use of a fed-batch process typically enables the use of a considerably higher amount of carbon and nitrogen source than is used in a batch process. Specifically, the amount of carbon and nitrogen source applied in a fed-batch process can be at least about two times higher than the highest amount applied in a batch process. This, in turn, leads to a considerably higher amount of biomass formed in a fed-batch process.

A further aspect of the present invention concerns the option of downstream processing of the fermentation broth. After the fermentation process is ended, the valuable product optionally may be recovered from the fermentation broth, using standard technology developed for the valuable compound of interest. The relevant downstream processing technology to be applied thereby depends on the nature and cellular localization of the valuable compound. First of all, the biomass is separated from the fermentation fluid using e.g. centrifugation or filtration. The valuable compound then is recovered from the biomass, in the case that the valuable product is accumulated inside or is associated with the microbial cells. Otherwise, when the valuable product is excreted from the microbial cell, it is recovered from the fermentation fluid.

The use of a chemically defined medium in the industrial fermentative production of a valuable compound of interest produces a large advantage in downstream processing, since the amount of byproducts is substantially lower than when complex media are used. In addition, the quality of the product is improved, since no undesired byproducts are coisolated with the compound of interest.

In still a further aspect of the present invention, a suitable strain for an industrial fermentation process using a chemically defined medium is identified.

A suitable microbial strain for an industrial fermentation process using a chemically defined medium may be any wild type strain producing a valuable compound of interest, provided that said wild type strain has a good growth performance on a chemically defined medium. In addition, a suitable microbial strain for an industrial fermentation process using a chemically defined medium may be a strain which is obtained and/or improved by subjecting a parent strain of interest to a classical mutagenic treatment or to recombinant DNA transformation, also with the proviso that the resulting mutant or transformed microbial strain has a good growth performance on a chemically defined medium. It will thereby depend on the growth performance of the parent strain on a chemically defined medium whether the resulting mutant or transformed strains should have an improved or a similar growth performance on a chemically defined medium as compared to that of the parent strain.

A microbial strain is understood to have a good growth performance on a chemically defined medium when said strain has a specific growth rate (*µ*) on a chemically defined medium which is ≥ 0.05 h⁻¹, preferably ≥ 0.1 h⁻¹, more preferably ≥ 0.2 h⁻¹, most preferably ≥ 0.4 h⁻¹. The growth performance of a microbial strain on a chemically defined medium is conveniently analyzed by fermentation of said strain in a chemically defined medium on a relatively small scale, e.g. a shake flask culture and/or a 10 L bench fermentation. It is preferred to include a 10 L bench fermentation, with a pH, temperature and oxygen concentration control, in the analysis of said growth performance.

In one embodiment of the invention, microbial strains which are capable of being fermented in a chemically defined medium are obtained and/or improved by subjecting a parent strain of interest to a classical mutagenic treatment using physical means, such as UV irradiation, or a suitable chemical mutagen, such as N-methyl-N'-nitro-N-nitrosoguanidine or ethylmethane sulfonate. In another embodiment of the invention, microbial strains which are capable of being fermented in a chemically defined medium are obtained and/or improved by subjecting a parent strain of interest to recombinant DNA technology, whereby the parent strain is transformed with a one or more functional genes of interest.

In general, the present invention envisages two groups of parent strains of interest to be subjected to classical mutagenesis and/or DNA transformation. In one embodiment of the invention, a parent strain of interest is selected from the group of strains which have a good growth performance on a chemically defined medium, but which need to be improved with regard to their production level of a compound of interest. In another embodiment of the invention, a parent strain of interest is selected from the group of strains which have a high production level of a compound of interest, but which have a relatively bad growth performance on a chemically defined medium. Microbial strains with a specific growth rate which is less than about 0.05 h⁻¹ are understood to have a relatively bad growth performance on a chemically defined medium.

Both processes, the classical mutagenic treatment as well as the DNA tranformation process, are followed by a screening of the resulting mutants or transformants for both their growth performance on a chemically defined medium as well as their production level of a compound of interest. Mutant strains or transformants are selected which have a good growth performance on a chemically defined medium and/or an improved production level of a compound of interest as compared to the parent strain.

It should be noted that some microbial strains, in particular industrial strains which already have been subjected to an extensive mutagenic treatment to improve production levels, may perform badly or may not grow at all in a chemically defined medium. Such a bad performance or lack of growth of a mutagenized strain may be caused by the fact that growth on a chemically defined medium never was applied as a criterion for selection of appropriate mutants. For instance, it is possible that a mutagenized strain possesses a mutation causing an unknown growth requirement (unknown auxotrophic mutation).

Microbial strains which are suitable for industrial fermentation using a chemically defined medium include filamentous and non-filamentous strains. For instance, microbial strains which are suitable for fermentation in a chemically defined medium include fungal strains, such as Aspergillus, Penicillium or Mucorales, yeast strains, such as Saccharomyces, Pichia, Phaffia or Kluyveromyces strains and bacterial strains, such as Actinomycetes. The use of chemically defined media according to the invention is especially advantageous for the industrial fermentation of filamentous microorganisms.

The process according to the invention using a chemically defined medium is suitable for the fermentative production on an industrial scale of any valuable compound of interest, including primary or secondary metabolites, pharmaceutical proteins or peptides, or industrial enzymes. Preferred valuable compounds are secondary metabolites, such as antibiotics or *β-*lactam compounds, especially β-lactam antibiotics.

Examples of strain-product combinations include A. *niger,* for instance A. *niger* CBS 513.88, for amyloglucosidase, *A. oryzae* for α-amylase, A. *terreus,* for instance A. *terreus* CBS 456.95, for lovastatin, *Mortierella alpina* for arachidonic acid or lipid containing arachidonic acid, *Mucor miehei* for protease, *P. chrysogenum,* for instance *P. chrysogenum* CBS 455.95 or other suitable strains, for β-lactam compounds (penicillin G or V), *Streptomyces clavuligerus,* for instance *S. clavuligerus* ATCC 27064, for clavulanic acid, *Pichia ciferrii,* for instance *P. ciferrii* NRRL Y-1031 F-60-10, for tetraacetyl-phytosphingosine, *Phaffia rhodozyma,* for instance *P. rhodozyma* CBS 6938, for astaxanthin, *Saccharopolyspora erythraea* for erythromycin, *K. lactis* for lactase, *Streptomyces natalensis* for natamycin.

The present invention also envisages the use of microbial strains which are transformed with one or more functional genes of interest, resulting in a transformed strain which may overexpress a product which already is produced by said strain, or resulting in a transformed strain which may express a product not naturally produced by said strain.

It is thereby left to the choice of the skilled person which strain will be selected for transformation, provided that said selected strain has a good growth performance on a chemically defined medium. For instance, a strain may be selected for transformation which already has been subjected to one or more mutagenic treatments. Alternatively, a non-mutagenized or wildtype strain may be selected. Next to analysis of the expression level of a desired compound, transformants obtained after transformation of a selected strain with one or more functional genes of interest should be analyzed for their growth performance on a chemically defined medium.

Examples of recombinant strains producing a product not naturally produced by said strain are:
**Streptomyces lividans,* for instance S. *lividans* TK21, containing an expression cassette enabling expression of glucose isomerase, the gene encoding glucose isomerase originating from e.g. *Actinoplanes missouriensis,*
**Penicillium chrysogenum,* for instance *P. chrysogenum* CBS 455.95, containing one or more expression cassettes enabling expression of an expandase, and optionally, a hydroxylase and/or an acetyltransferase, the genes encoding expandase, hydroxylase and acetyltransferase originating from e.g. *Acremonium chrysogenum* or *Streptomyces clavuligerus,* enabling production of cephalosporin compounds, such as 7-ADCA or 7-ACA, using adipic acid (see EP 532341) or 3-carboxymethylthio-propionic acid (see WO95/04148) as a side chain precursor,
**Aspergillus niger,* for instance *Aspergillus niger* CBS 513.88, containing an expression cassette enabling expression of human lactoferrin (see WO93/22348) or bovine chymosin.
**Kluyveromyces lactis,* containing an expression cassette enabling expression of bovine chymosin or phospholipase A₂, insulin or recombinant human albumin.

Examples of recombinant strains overproducing an enzyme already produced by said strain are:
*A. *niger,* for instance A. *niger* CBS 513.88, containing an expression cassette enabling overexpression of phytase (see EP 420358) or endoxylanase I (see EP 463706).

The present invention is exemplified by an industrial scale fermentation process using a chemically defined medium for the production of glucose isomerase by a recombinant Streptomyces strain, and by the advantageous use of chemically defined media for jarge scale Penicillium fermentation as compared to complex media.

Additional examples are directed to chemically defined media which can be used to measure the growth performance and productivity of a strain of interest when grown in such a medium on a small scale, in order to identify microbial strains which are suitable for fermentative production of a valuable compound on an industrial scale in a chemically defined medium.

### Short description of the Figures

Figure 1. Outline of pWGx.GIT.
Figure 2. Development of total glucose isomerase produced during fermentation.

### Example 1

### Industrial production of glucose isomerase using Streptomyces lividans

### Construction of a Streptomyces strain producing glucose isomerase

The glucose isomerase gene of *Actinoplanes missouriensis* was originally cloned as a DNA fragment of 5.0 kb in *E.coli* K12 strain JM101.

A 1.7 kb fragment internal to the 5.0 kb fragment, was found to represent the complete coding sequence of A. *missouriensis* glucose isomerase and its upstream regulatory region (see also Amore and Hollenberg (1989), Nucl. Acids Res. 17, 7515).

A glucose isomerase mutant exhibiting enhanced thermostability was obtained by changing within the glucose isomerase gene the triplet AAG encoding lysine at position 253 of the glucose isomerase protein into CGG encoding arginine (Quax et al. (1991), Bio/Technology 9, 738-742).

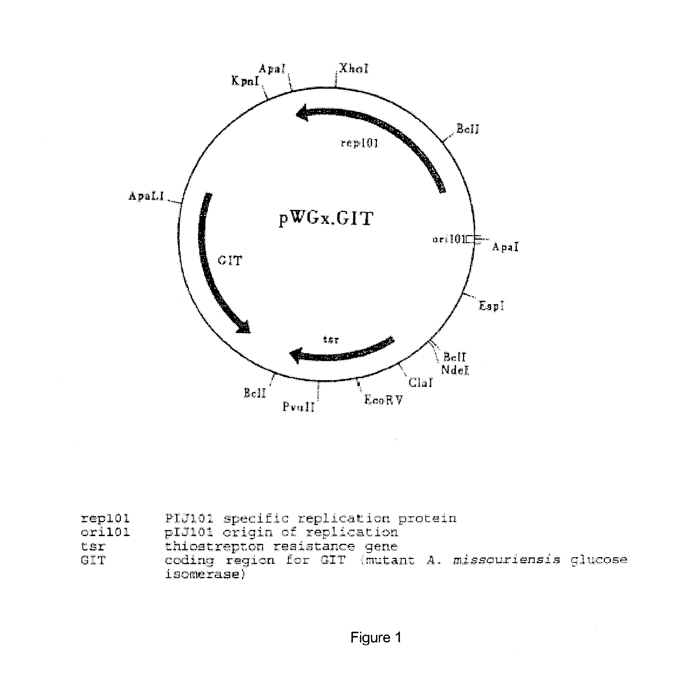

For cloning in Streptomyces plasmid plJ486 (Ward et al. (1986), Mol. Gen. Genet. 203, 468-478) was used as a vector. The 1737 basepair *A.missouriensis* DNA fragment encoding glucose isomerase was combined with the large Pstl DNA fragment of plJ486. The resulting plasmid, called pWGx.GIT contained essentially the replication region of plasmid plJ101, the thiostrepton resistance gene, and the A. *missouriensis* DNA fragment encoding GIT. A schematic map of pWGx.GIT is given in Figure 1.

The glucose isomerase producing strain was constructed by transformation of *Streptomyces lividans* strain TK21 (Hopwood et al. (1985), Genetic Manipulation of Streptomyces: A Laboratory Manual. The John Innes Foundation, Norwich, England) with plasmid pWGx.GIT.

### Industrial production of glucose isomerase

A working cell bank of a production strain constructed as mentioned in Example 1 was prepared by picking a thiostrepton-resistant colony and growing it in 20 ml Tryptone Soytone Broth containing thiostrepton (50 mg/L) in a 100 ml shake flask at 30°C for 40-48 hours and shaking at 280 rpm.

Mycelium equivalent to 1 ml of the working cell bank (fresh or stored as frozen mycelium at -80 °C) is inoculated in 100 ml inoculum growth medium in a 500 ml shake flask, containing 16 g/L Bactopeptone (Difco 0123/01), 4 g/L Bacto soytone (Difco 0436/01), 20 g/L Casein hydrolysate (Merck 2239), 10 g/L dipotassiumphosphate.3aq (Merck, Anal. Reagent), 16.5 g/L glucose.1aq, 0.6 g/L soybean oil and 50 mg/L thiostrepton. The pH of the medium is adjusted to 7.0 with sodiumhydroxide and/or sulphuric acid prior to sterilization. Glucose and thiostrepton are added separately after sterilization, Thiostrepton is dissolved in DMSO in a concentration of 50 g/l and filter sterilized over a 0.2 *µ*m Nalgene filter. The culture is grown for 24 hours at 30 °C in a incubator shaker at 280 rpm.

50 ml of the full grown culture is transferred to 6 L of the second phase inoculum growth medium having a composition similar to the previous mentioned medium, except for a double glucose concentration (33 g/L glucose.1aq), extra antifoam (SAG5693, 0.6 g/L; a silicon antifoam from Basildon company) and without thiostrepton. Glucose is again separately sterilized in 50% solution and added after sterilization of the medium (60 minutes, 121 °C). The culture is grown for 36 hours in a sterilized bubble column aerated with 840 L sterile air/h with a nozzle containing 4 holes with a diameter of 2 mm and the temperature is maintained at 22 °C. Alternatively, this phase may be carried out in shakeflasks (e.g. 12x 500 ml medium in 2 L baffled Erlenmeyer flasks) with similar inoculation ratios and shaken at 280 rpm in an orbital shaker incubator.

The full-grown culture is transferred aseptically to an inoculum fermentor containing 4.5 m³ of inoculum medium containing 16.3 kg citric acid.1aq, 70.8 gr ferro-sulphate.7aq, 109 gr zinc-sulphate.7aq, 109 gr manganese-sulphate.1aq, 32.7 gr cobalt-dichloride.6aq, 5.45 gr disodium-molybdate.2aq, 5.45 gr boric acid, 5.45 gr copper-sulphate.5aq, 10.9 kg di-ammonium-sulphate, 10.9 kg magnesium-sulphate.7aq, 463 gr calcium-chloride.2aq, 1090 gr soybean-oil, 21.8 kg monopotassium-phosphate and 139 kg glucose.1aq and 5.9 kg yeast extract (brewers yeast with 10% Kjeldahl nitrogen on dry weight basis). The medium is prepared as follows: all components except glucose are charged in the indicated sequence in approximately 2700 L tapwater. The pH is set at 4.5 with sodium hydroxide and/or phosphoric acid and the medium is sterilized at 122 °C for 60 minutes. The glucose is sterilized in 1000 L of water at pH 4.5 for 60 minutes at 122 °C in a separate vessel. After cooling down of both portions, the glucose is transferred aseptically to the inoculum-vessel. After mixing of both portions the pH is set at 7.0 with ammonia and the volume is adjusted with sterile water to 4.5 m³. The temperature of the fermentation is controlled at 30 °C and the fermentor is aerated at 0.5-1.0 vvm while the pH is maintained at 7.0 +/- 0.1 with gaseous ammonia and the overpressure is maintained at 1.3-1.4 bar. Foaming is controlled if necessary with a sterilized mixture of soybean oil and a silicon antifoam, like SAG5693, in a ratio of 3:1. The oxygen concentration is maintained above 25% of air-saturation by adjusting the stirrer speed (0.5 to 3 Kw/ m³). The culture is transferred to the main fermentation before all glucose is consumed (as in all previous growth phases) and before the oxygen uptake rate exceeds a level of 30 mmol/l broth volume.h.

The main fermentation medium contains 245.1 kg citric acid.1aq, 1062 gr ferro-sulphate.7aq, 1634 gr zinc-sulphate.7aq, 1634 gr manganese-sulphate.1aq, 490 gr cobalt-dichloride.6aq, 82 gr disodium-molybdate.2aq, 82 gr boric acid, 82 gr copper-sulphate.5aq, 163.4 kg di-ammonium-sulphate, 163.4 kg magnesium-sulphate.7aq, 6.94 kg calcium-dichloride.2aq, 16.3 kg soybean oil, 327 kg monopotassium-phosphate, 880 kg Brewers yeast extract (10% Kj-N on dry weight basis) and 556 kg glucose.1aq. The medium is prepared as described for the inoculum fermentation (glucose is sterilized separately). For glucose a DE-95 sugar syrup may be used alternatively. The volume of the medium prior to inoculation is 65 m³ after the pH is corrected to 7.0 with ammonia.

A glucose feed is prepared at 275 to 550 g glucose/L feed-solution, either as glucose.1aq or as glucose equivalents from a >90-DE-syrup. The pH is adjusted to 4.5-5.0 with a phosphoric acid solution. Sterilization is done either batch (122 °C, 45 minutes) or continuously via a heatshock or filterset.

The main fermentation is controlled at 30 °C +/- 0.5 and pH 7.0 +/- 0.1 (by means of a pH control using ammonia and phosphoric acid). The airflow is set at 0.5-1.5 vvm, preferably 0.7 vvm, overpressure is 0.5-1.5 bar and the fermentor is stirred with Rushton turbines at an intensity of 0.5 to 3 Kw/m³ in order to prevent the oxygen concentration to go below 0.2 mmol/L, measured at the bottom stirrer height. The glucose feed is started when a sudden drop in oxygen uptake rate occurs, and the dissolved oxygen concentration increases, as well as the pH which comes from 6.9 to 7.1. The glucose concentration in the broth should be <<0.2 g/L at this point in time.

The glucose feed rate is equivalent to 93 kg glucose/h initially increasing linearly to 186 kg/h at 64 hours after feed start. After 100 feeding hours at 186 kg/h the feed rate is increased to 298 kg glucose/h until approximately 200 feeding hours.

Foaming is controlled by dosing sterile soybean-oil at 5.5 kg/hr or alternatively in shots of 45 kg every 8 hours during the first 100 hours of the fermentation. If necessary further foam control is done with a mixture of soybean oil and a silicon antifoam like SAG471 (silicon antifoam from Basildon) in a ratio 3:1.

The ammonia concentration is maintained between 750 and 1500 mg/L by measuring every 12 hours and adding sterile ammonium sulphate in portions equivalent to 500 mg ammonia/L, as soon as the level has dropped below 1000 mg/L.

The phosphate concentration in the culture filtrate should be maintained higher than 500 mg PO₄ /L by adding sterile monopotassiumphosphate in portions equivalent to 500 mg/L.

The production of glucose isomerase can be measured as protein harvested and purified from the broth followed by protein determination methods known in the art or assayed in an enzymatic assay applied on a stabilized broth sample. The broth samples are stabilized by weighing 2 gr of broth and adding 5 ml of stabilizer solution containing 12 g/L tris-hydroxymethylaminomethane and 2.4 g/L CoCl₂.6aq which is subsequently heated for 30 minutes at 73 °C. After cooling down 0.42 ml of stabilized sample is mixed with 0.8 ml glucose solution (containing 27.25 g/L Tris/HCl buffer pH 8.2, glucose 67.56 g/L, MgCl₂.6aq, 22,33 g/L Na₂-EDTA.2aq and 5 mg/L Triton X-100) and incubated at 60 °C. The activity is determined by measuring the conversion rates of glucose to fructose and expressed as GU/g. (1 GU is the amount of enzyme required for the formation of 1 *µ*mole fructose/min.) Using the specific activity of 12 Units per mg protein, the amount of protein per kg broth can be determined.

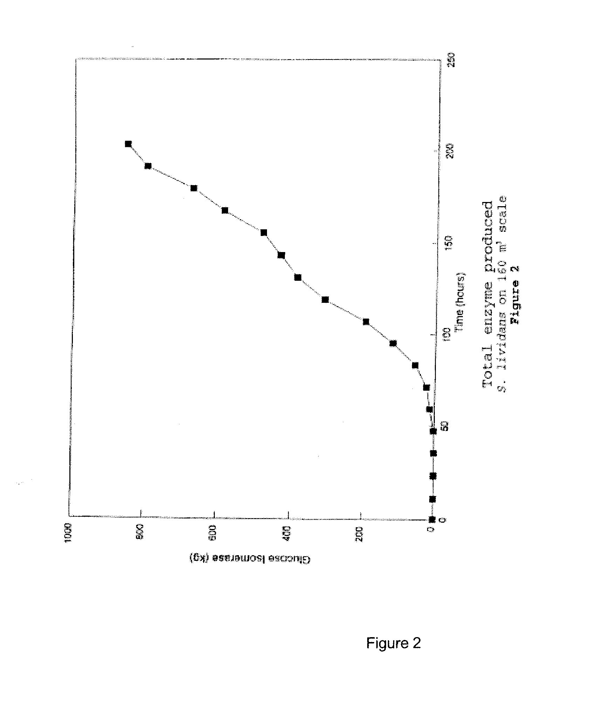

In Figure 2 the total amount of enzyme produced is indicated.

As is demonstrated in this example 850 kg of the enzyme can be manufactured in one fed batch production run.

### Example 2

### Production of penicillin V

Conidiospores of a *P. chrysogenum* CBS 455.95 (or another suitable strain derived from Wisconsin 54.1255 by mutation and selection for higher productivity, preferably in the recipe as stated below) are inoculated at 10E5 - 10E6 conidia/ml in a production medium containing (g/l): glucose.H₂O, 5; lactose.H₂O, 80; (NH₂)₂CO, 4.5; (NH₄)₂SO₄, 1.1; Na₂SO₄, 2.9; KH₂PO₄, 5.2; K₂HPO₄.3H₂O, 4.8; trace elements solution (citric acid.H₂O, 150; FeSO₄.7H₂O, 15; MgSO₄.7H₂O, 150; H₃BO₃, 0.0075; CuSO₄.5H₂O, 0.24; CoSO₄.7H₂O, 0.375; ZnSO₄.7H₂O, 1.5; MnSO₄.H₂O, 2.28; CaCL₂.2H₂O, 0.99), 10 (ml/l); 10% potassium phenoxyacetate solution, pH 7, 75 (ml/l). (pH before sterilization 6.5).

The culture is incubated at 25 °C in an orbital shaker at 280 rpm for 144-168 hours. At the end of the fermentation, the mycelium is removed by centrifugation or filtration and the amount quantified, and the medium is assayed for penicillin formed by HPLC methods well known in the art.

### Example 3

### Large scale Penicillium fermentation

### with complex and defined media

*Penicillium chrysogenum* Wisconsin 54.1255 was optimized for growth and penicillin production on a chemically defined medium by mutation and selection on defined media as described in Example 2. Fed batch fermentations were carried out on 60 m³-scale with a complex medium as described by Hersbach at al. (Biotechnology of Industrial Antibiotics pp 45-140, Marcel Dekker Inc. 1984, Table 4, Medium B, including the salts as mentioned under Medium A) containing 50 kg/m³ Corn Steep Solids. Parallel to that, a fermentation was carried out in a defined medium as given in Example 2, where the dosages were doubled because of the high cell density character of these fed batch fermentations while lactose and ureum were omitted. Glucose was fed to the fermentor keeping the glucose concentration below 2 g/L to avoid glucose repression. Ammonium, di-ammonium-sulphate and phenyl-acetic acid were fed to the fermentor in order to control the pH and the concentrations of ammonium, sulphate and phenylacetic acid in the desired ranges (Hersbach 1984).

Since oxygen transfer is an important parameter determining the economical feasibility of an industrial fermentation process, the performance of the above fermentation processes was analyzed by determining the extent of oxygen transfer in each process.

A good measure for the oxygen transfer obtained in a fermentation process is the relative k_{L}a value as determined within one system. k_{L}a is defined as the oxygen transfer coefficient and is calculated according to van 't Riet and Tramper (Basic Biorector Design, Marcel Dekker Inc. (1991), pp. 236-273).

The oxygen transfer capacity calculated as the k_{L}a-value was found to be between 10 and 20% higher in the chemically defined medium than in the complex medium, during the main part of the fermentation.

### Example 4

### Production of 7-ADCA

The process as described in Example 2 is modified by using a *P*. *chrysogenum* CBS 455.95 (or another suitable strain derived from Wisconsin 54.1255 by mutation and selection for higher productivity, preferably in the recipe as stated below) which is transformed with an expandase expression cassette wherein the expandase coding region is provided with the IPNS promoter, as described in EP 532341, and using a 10% potassium adipate solution instead of phenoxyacetate and using a modification of the above medium containing 400 ml of a 10% potassium adipate solution, pH 5.5 instead of phenoxyacetate (pH of medium before sterilization 5.5 instead of 6.5).

The resulting adipoyl-7-ADCA subsequently is converted to 7-ADCA using the enzymatic deacylation process substantially as described in Example 4 or 5 of WO95/04148.

### Example 5

### Production of lovastatin

Conidiospores or *Aspergillus terreus* strain CBS 456.95 (or strains derived thereof by mutation and selection for higher productivity, preferably in either of the recipes as stated below) are inoculated at 10E5-10E6 conidia/ml in a production medium containing (g/l): dextrose, 40; CH₃COONH₄, 2.2; Na₂SO₄, 4; KH₂PO₄, 3.6; K₂HPO₄.3H₂O, 35.1; trace elements solution (*vide supra,* Ex. 2), 10 (ml/l).

The culture is incubated at 28 °C in an orbital shaker at 220 rpm for 144-168 hours. At the end of the fermentation, the mycelium is removed by centrifugation or filtration and the amount quantified, and the medium is assayed for lovastatin formed by HPLC methods well known in the art.

### Example 6

### Production of clavulanic acid

*Streptomyces clavuligerus* strain ATCC 27064 or a mutant thereof is inoculated in a preculture medium consisting of (g/l): (NH₄)2SO₄, 2.75; KH₂PO₄, 0.5; MgSO₄.7H₂O, 0.5; CaCl₂.2H₂O, 0.01; 3-(N-morpholino), propanesulfonic acid, 21; glycerol, 19.9; sodium succinate, 5.5; trace elements solution (ZnSO₄.7H₂O, 2.8; ferric ammonium citrate, 2.7; CuSO₄.5H₂O,0.125; MnSO_{4.}H₂O, 1; CoCl₂.6H₂O, 0.1; Na₂B4O7.10H₂O, 0.16; Na₂MoO₄.2H₂O, 0.054), 0.06 (ml/l).

The culture is incubated in an orbital shaker at 220 rpm at 28 °C for 48-72 hours and used to inoculate 20 volumes of production medium containing (g/l): (NH₄)2SO₄, 2; asparagine, 4; KH₂PO₄, 1.5; MgSO₄.7H₂O, 0.5; CaCl₂.2H₂O, 0.01; 3-(N-morpholino), propanesulfonic acid, 21; glycerol, 19.9; sodium succinate, 5.5; trace elements solution (*vide supra*)*,* 0.06 (ml/l); FeSO₄.7H₂O, 0.5; MnCl₂.4H₂O, 0.1; ZnSO₄.7H₂O, 0.1.

The incubation is continued for 144 hours, preferably in a 500 ml Erlenmeyer flask with baffles, containing 50 ml of culture volume. At the end of the fermentation, the mycelium is removed by centrifugation or filtration and the amount quantified, and the filtrate is assayed by HPLC methods well known in the art.

### Example 7

### Production of amyloglucosidase

*Aspergillus niger* strain CBS 513.88 or a mutant thereof is inoculated at 10⁵ - 10⁶ conidiospores/ml in a germination medium consisting of (g/l): K₂HPO₄.3H₂O, 0.75; KH₂PO₄, 6.6; Na3citrate.3H₂O, 5.3; citric acid.H₂O, 0.45; glucose.H₂O, 25; (NH₄)2SO₄, 8; NaCl, 0.5; MgSO₄.7H₂O, 0.5; FeSO₄.7H₂O, 0.1; ZnSO₄.7H₂O, 0.05; CaCl₂, 0.005; CuSO₄.5H₂O, 0.0025; MnSO₄.4H₂O, 0.0005; H₃BO₃, 0.0005; Na₂MoO₄.2H₂O, 0.0005; EDTA, 0.13; Tween80, 3. If necessary, 50*µ*g/ml arginine and/or proline may be added to improve germination.

The culture is incubated in an orbital shaker for 48-72 hours at 33°C, 295 rpm and then used to inoculate 10-20 volumes of a production medium consisting of (g/l): KH₂PO₄, 1-5; NaH₂PO₄.H₂O, 0.5; Na3citrate.3H₂O, 53; citric acid.H₂O, 4.05; dextrose polymers 70; (NH₄)2SO₄, 8; (NaCl, MgSO₄.7H₂O, FeSO₄.7H₂O, ZnSO₄.7H₂O, CaCl₂, CuSO₄.5H₂O, MnSO₄.4H₂O, H₃BO₃, Na₂MoO₄.2H₂O, EDTA): same as germination medium.

The incubation is continued for 96 hours, preferably in a 500 ml Erlenmeyer flask containing 100 ml of medium. At the end of the fermentation, the mycelium is removed by centrifugation or filtration and the amount quantified, and the filtrate is assayed for amylolytic activity.

### Example 8

### Production of astaxanthin

*Phaffia rhodozyma* strain CBS 6938 or a mutant thereof is inoculated in 25 ml of a preculture medium containing (g/l) yeast extract, 10; peptone, 20; glucose, 20.

The culture is incubated for 72 hours at 20°C, in a 100 ml Erlenmeyer flask with baffle, in an orbital shaker at 275 rpm.
1 ml of the preculture is then used to inoculate 100 ml of a production medium containing (g/l): glucose, 30; NH₄Cl, 4.83; MgSO₄.7H₂O, 0.88; NaCl, 0.06; CaCl₂.6H₂O, 0.15; trace elements solution (citric acid.H₂O, 50; (NH₄)2Fe(SO₄)2.6H₂O, 90; ZnSO₄.7H₂O, 16.7; CuSo4.5H₂O, 2.5; MnSO₄.4H₂O, 2; H₃BO₃, 2; Na₂MoO₄.2H₂O, 2; Kl, 0.5; in 0.4N H₂SO₄), 0.3 (ml/l); vitamins solution (myo-inositol, 40; nicotinic acid, 2; Ca-D-pantothenate, 2; vitamin B1, 2: p.aminobenzoic acid, 1.2; vitamin B6, O.2; biotin 0.008; in 0.07N H₂SO₄) 1-5 (ml/l); pluronic, 0.04; KH₂PO₄, 1; potassium hydrogen phthalate, 20 (pH before sterilization 5.4).

The incubation is continued for 96 hours preferably in a 500 ml Erlenmeyer flask with baffle. At the end of the fermentation, the astaxanthin content of the biomass (amount quantified) is determined by solvent extraction and measuring the optical density of the extract at 470 - 490 nm.

### Example 9

### Production of arachidonic acid

One 1 ml vial of a suspension of *Mortierella alpina* strain ATCC 16266 stored at -80 °C is opened aseptically and the content is used to inoculate 500 ml of a production medium containing (g/l):
glucose, 70; yeast extract 0.5; NH₄NO₃ 3.0; KH₂PO₄7.2; MgSO₄.7H₂O 1.5; trace elements solution (citric acid.H₂O 50; (NH₄)₂Fe(SO₄)2.6H₂O, 90; ZnSO₄.7H₂O, 16.7; CuSo₄.5H₂O, 2.5;
MnSO₄.4H₂O, 2; H₃BO₃, 2; Na₂MoO₄.2H₂O, 2; Kl, 0.5; in 0.4N H₂SO₄), 0.3 (ml/l); (pH before sterilization 7.0).

The culture is incubated in a 2 litre shake flask with baffles, at 25 °C during 72 hours in a orbital shaker at 250 rpm. At the end of the fermentation, the amount of biomass and the arachidonic acid content of the biomass is determined, after centrifugation, freeze drying and solvent extraction, by GC methods well known in the art.

### Example 10

### Production of erythromycin

*Saccharopolyspora erythraea* strain NRRL2338 or a mutant thereof (selected for increased productivity, preferably in the recipe as stated below) is inoculated in 25 ml of a preculture medium containing (g/l):
Soluble starch, 15; NaCl, 5; Soy bean meal, 15; CaCO₃, 10; Yeast-extract, 5, Cornsteep solids, 5; C_{O}Cl₂.6H₂O, 670 µl of a 1 g/l solution.

The culture is incubated in a 100 ml shake flask without baffles at 32-34 °C for 3 days at 250 RPM in a shaker-incubator.

0.4 ml of the culture is inoculated in 25 ml of a sterile production medium containing (g/l):
Citric acid.H₂O, 2; (NH₄)₂SO₄ 2; MgSO₄.7H₂O, 2; CaCl₂.2H₂O, 0.085; KH₂PO₄, 0.25; HEPES (= N-(2-hydroxyethyl)piperazine-N'-(2-ethanesulphonic acid)), 5; Glucose, 1.5; Soluble starch, 20; Soy-oil, 0.4 ; trace elements solution (gram in 250 ml distilled water: Citric acid.H2O, 62.5, FeSO₄.7H₂O, 0.8215; CuSO₄.5H₂O, 0.0625;, CoCl₂.H₂O, 0.375;, H₃BO₃, 0.0625; ZnSO₄.7H₂O, 1.25; MnSO₄.H₂O, 1.25; Na₂MoO₄.2H₂O, 0.0625), 3.6 ml/l. pH=7.0. To each flask, 0.25 ml of n-propanol is added.

The culture is incubated in a 100 ml shake flask with baffles at 32-34 °C during 5 days in a shaker-incubator at 300 RPM. At the end of the fermentation, the broth is centrifuged and the amount of biomass quantified. The erythromycin content of the supernatant is measured by HPLC methods known in the art.

### Example 11

### Production of β-carotene

A spore suspension of *Blakeslea trispora* CBS 130.59 is used to inoculate 114 ml of preculture medium (yeast extract 10 g/l; peptone 20 g/l; glucose 20 g/l) in a 500 ml shake flask without baffles. The preculture is incubated for 42 h on a rotary shaker at 250 rpm at 26°C. The biomass is harvested by filtration, and washed 3 times with 100 ml sterile demineralized water to remove the components of the preculture medium. Subsequently the biomass is homogenized by blendering, until only small fragments remain, and resuspended in 40 ml demineralized water.

The production medium is prepared in 100 ml portions in 500 ml baffled shake flasks. The composition of the production medium is as follows (in g/l): glucose, 40; asparagine monohydrate, 2; KH₂PO₄, 0.5; MgSO₄.7H₂O, 0.25. In addition a trace element solution is added (0.3 ml/l) with the following composition (in g/l): citric acid.H₂O, 50; (NH₄)₂Fe(SO₄)₂.6H₂O, 90; ZnSO₄.7H₂O, 16.7; CuSO₄.5H₂O, 2.5; MnSO₄.4H₂O, 2; H₃BO₃, 2; Na₂MoO₄.2H₂O, 2; Kl, 0.5; in 0.4N H₂SO₄. Before sterilization the medium pH is adjusted to 6.2. The flasks are sterilized for 20 minutes at 120°C, and after sterilization 0.05 ml of a 1 mg/ml solution of thiamin hydrochloride in demineralized water (sterilized by filtration) is added.

The production cultures are inoculated with 0.5 to 10 ml of the suspension of fragmented mycelium prepared above. The cultures are incubated for 139 h on a rotary shaker (250 rpm; 26°C). The fungal biomass is harvested by filtration, washed with demineralized water to remove medium components and quantified.

The amount of β-carotene produced is determined by acetone extraction and measuring the extinction at 450 nm of the acetone fraction in a spectrophotometer.

## Claims

1. A process for the production of a valuable compound, comprising the steps of:
* fermentation of a microbial strain on an industrial scale in a fermentation medium which is a chemically defined medium essentially composed of chemically defined constituents, and
* recovery of the valuable compound from the fermentation broth.

2. The process of claim 1, wherein the chemically defined medium contains an essentially small amount of a complex carbon and/or nitrogen source.

3. The process of claim 1 or 2, wherein the chemically defined constituents of the chemically defined medium comprise a carbon source selected from the group consisting of carbohydrates such as glucose, lactose, fructose, sucrose, maltodextrins, starch and inulin, glycerol, vegetable oils, hydrocarbons, alcohols such as methanol and ethanol, organic acids such as acetate and higher alkanoic acids, and a nitrogen source selected from the group consisting of urea, ammonia, nitrate, ammonium salts such as ammonium sulphate, ammonium phosphate and ammonium nitrate, and amino acids such as glutamate and lysine.

4. The process of claim 3, wherein the carbon source is glucose and the nitrogen source is ammonia and/or an ammonium salt.

5. The process of any one of the claims 1 to 4, wherein fermentation occurs via a batch, a repeated batch, a fed-batch, a repeated fed-batch or a continuous fermentation process.

6. The process of claim 5, wherein fermentation occurs via a fed-batch process.

7. The process of claim 6, wherein a carbon and/or a nitrogen source is fed to the process.

8. The process of claim 7, wherein the carbon source is glucose and the nitrogen source is ammonia and/or an ammonium salt.

9. The process of any one of the claims 1 to 8, wherein the valuable compound is a pharmaceutical protein or peptide, a primary or a secondary metabolite, or an industrial enzyme.

10. The process of any one of the claims 1 to 9, wherein the microbial strain is a filamentous microbial strain.

11. The process of claim 10, wherein the filamentous strain is a fungus or a yeast.

12. The process of claim 11, wherein the fungus is a Mucorales strain, preferably a Mortierella strain.

13. The process of claim 12, wherein the Mucorales strain is *Mortierella alpina* and the valuable compound is a lipid comprising arachidonic acid, optionally, wherein the lipid comprising arachidonic acid is a triglyceride.

14. A method for preparing and/or improving a microbial strain producing a valuable compound of interest which is capable of being fermented on an industrial scale in a chemically defined medium, comprising the steps of:
* subjecting a suitable parent strain to a mutagenic treatment selected from the group of physical means and chemical mutagens, and/or to DNA transformation,
* screening the resulting mutants and/or transformants for their growth performance on a chemically defined medium and their production level of said valuable compound of interest,
* selecting mutants and/or transformants which have a good growth performance on a chemically defined medium and/or an improved production level of said valuable compound of interest as compared to said parent strain,
and, optionally, wherein
(i) the parent strain is selected from the group consisting of strains which have a good growth performance on a chemically defined medium, but which need to be improved on production level; or
(ii) the parent strain is selected from the group consisting of strains which have a high production level of a desired compound but a relatively bad growth performance on a chemically defined medium.

15. Use of a chemically defined fermentation medium for the production of a valuable compound by fermentation of a microbial strain on an industrial scale.

16. The process of claim 9, wherein the valuable compound is a secondary metabolite.

17. The process of claim 16, wherein the secondary metabolite is a β-lactam compound.

18. The process of claim 9, wherein the valuable compound is an enzyme.

19. The process of claim 11, wherein the yeast is *Phaffia rhodozyma* and the valuable compound is astaxanthin.

20. The process of claim 11, wherein the fungus is an Aspergillus strain.

21. The process of claim 20, wherein the fungus is *Aspergillus terreus* and the valuable compound is lovastatin.

22. The process of claim 11, wherein the fungus is a Penicillium strain.

23. The process of claim 22, wherein the fungus is *Penicillium chrysogenum* and the valuable compound is a β-lactam compound.

24. The process of claim 12, wherein the Mucorales strain is a Blakeslea strain.

25. The process of claim 24, wherein the Mucorales strain is *Blakeslea trispora* and the valuable compound is β-carotene.

26. The process of claim 10, wherein the filamentous strain is a bacterium.

27. The process of claim 26, wherein the bacterium is an Actinomycete.

28. The process of claim 27, wherein the Actinomycete is a Streptomyces strain and the valuable compound is glucose isomerase.

29. The process of claim 27, wherein the Actinomycete is *Streptomyces clavuligerus* and the valuable product is clavulanic acid.

30. The process of claim 27, wherein the Actinomycete is *Saccharopolyspora erythraea* and the valuable comopund is erythromycin.
